(19) Europäisches Patentamt
European Patent Office
Office européen des brevets

(11) Veröffentlichungsnummer: **0 260 560**
**B1**

(12) # EUROPÄISCHE PATENTSCHRIFT

(45) Veröffentlichungstag der Patentschrift: **16.05.90**

(51) Int. Cl.⁵: **C07D 277/32**

(21) Anmeldenummer: **87113029.0**

(22) Anmeldetag: **07.09.87**

(54) **Verfahren zur Herstellung von 2-Chlor-5-chlormethylthiazol.**

(30) Priorität: **17.09.86 DE 3631538**

(43) Veröffentlichungstag der Anmeldung: **23.03.88 Patentblatt 88/12**

(45) Bekanntmachung des Hinweises auf die Patenterteilung: **16.05.90 Patentblatt 90/20**

(84) Benannte Vertragsstaaten: **BE CH DE FR GB IT LI NL**

(56) Entgegenhaltungen: **EP-A- 0 192 060**

(73) Patentinhaber: **BAYER AG, D-5090 Leverkusen 1 Bayerwerk(DE)**

(72) Erfinder: **Beck, Gunther, Dr., Am Mittelberg 19, D-5090 Leverkusen 1(DE)**
Erfinder: **Heitzer, Helmut, Dr., Hoehenstrasse 84, D-5090 Leverkusen 3(DE)**

EP 0 260 560 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung der bekannten Verbindung 2-Chlor-5-chlormethylthiazol.

Die Verbindung 2-Chlor-5-chlormethylthiazol ist aus der veröffentlichen europ. Patentanmeldung Nr. 192 060 bekannt. Siehe dort beispielsweise Seite 81, Zeile 13.

Über die Herstellung von 4-Halogenmethylthiazolen wird auf der Seite 93, Zeilen 1 bis 24 folgendes aufgeführt:

"4-Halogenomethylthiazole lassen sich direkt synthetisieren, beispielsweise durch Reaktion von Dihalogenacetonen mit Thioacetylamiden wie Thioacetamid (J. Amer. Chem. Soc. 56, S. 470-471 und ibid. 73, S. 2936).

5-Halogenomethylthiazole lassen sich erhalten durch Reaktion eines Thiacylamids mit α-Chloro-α-formylethylacetat, Reduktion des erhaltenen 5-Ethoxycarbonylthiazols mit Lithiumaluminiumhydrid in üblicher Weise und Halogenierung des erhaltenen 5-Hydroxymethylthiazols. 5-Chlormethyl-2-methylthiazol, das in Zh. Obshch. Khim. 32, S. 570-575, und in J. Amer. Chem. Soc. 104, S. 4461-4465, beschrieben ist, ist ein gutes Beispiel.

Die Reaktion von Thioharnstoff an Stelle des Thioacylamids vermag 2-Amino-4-chloromethyl- oder 2-Amino-5-chloromethylthiazol zu liefern, und über eine Diazotierung kann weiterhin ein Halogen-Atom etc. eingeführt werden. Dieses Halogen ist aktiv und kann mit Hilfe eines Natriumalkoxids in eine 2-Alkoxy-Gruppe umgewandelt werden (JP-OS 5972/1979 und J. Chem. Soc. Perkin I, 1982, S. 159-164)."

Die oben genannten Verfahren sind arbeitsaufwendig und meist nur mit einer sehr geringen Ausbeute verbunden.

Es wurde gefunden, daß man 2-Chlor-5-chlormethylthiazol I

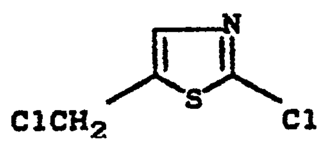

(I)

in einfacher Weise dadurch erhält, daß man
pro Mol Allylisothiocyanat (= Allylsenföl) der Formel

$CH_2$=CH-$CH_2$-NCS(II)

2 bis 20 Mol Chlor bei Temperaturen von 0°C bis 150°C gegebenenfalls in einem unter den Reaktionsbedingungen inerten Verdünnungsmittel einwirken läßt.

Es ist als ausgesprochen überraschend zu bezeichnen, daß gemäß der erfindungsgemäßen Umsetzung 2-Chlor-5-chlormethylthiazol herstellbar ist, da es bekannt war, daß aliphatische und cycloaliphatische Senföle durch Chlorierung in 70 bis 95 %iger Ausbeute in Isocyaniddichloride übergeführt werden (vgl. E. Kühle, B. Anders und G. Zumach: Isocyaniddihalogenid-Synthesen, Angewandte Chemie 79, 663 (1967)).

Das Reaktionsschema der erfindungsgemäßen Umsetzung kann wie folgt verdeutlicht werden:

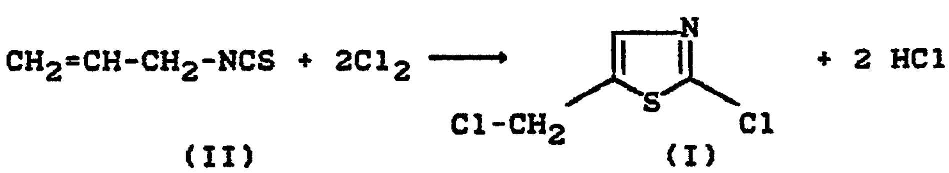

(II) (I)

Als Chlorierungsmittel werden Chlor und bei den Reaktionsbedingungen chlorabspaltende Verbindungen verstanden, wie z.B. Sulfurylchlorid, welches gemäß der Bruttogleichung

$SO_2Cl_2 \rightarrow SO_2 + Cl_2$

elementares Chlor abspaltet. In der Folge soll unter dem Begriff Chlor sowohl das von vornherein eingesetzte elementare Chlor als auch das unter den Reaktionsbe dingungen aus chlorabspaltenden Verbindungen freigesetzte Chlor verstanden werden.

Wie aus der Formelgleichung der Umsetzung von Allylisothiocyanat (II) mit $Cl_2$ zum Erhalt von 2-Chlor-5-chlormethylthiazol (I) hervorgeht, müssen zur Erzielung einer vollständigen Umsetzung mindestens 2 Mol Chlor pro Mol Allylsenföl eingesetzt werden. Im allgemeinen werden bevorzugt große Überschüsse an Chlorgas eingesetzt, die bis zu 20 Mol $Cl_2$ pro Mol (II) betragen können. Bevorzugt arbeitet man mit 5 bis 15 Mol $Cl_2$ pro Mol (II).

Zweckmäßig führt man die Chlorierungsreaktion in einem unter den Reaktionsbedingungen inerten Verdünnungsmittel durch. Als solche Verdünnungsmittel seien genannt: Methylenchlorid, Chloroform, Tetrachlormethan, Tetrachlorethan, Pentachlorethan, Trichlorethylen, Tetrachlorethylen. Bevorzugt ist Chloroform.

Im Falle von Sulfurylchlorid als Chlorierungsmittel kann die Umsetzung auch in Abwesenheit eines inerten Verdünnungsmittels erfolgen.

Die Chlorierungsreaktion kann im Temperaturbereich von 0°C bis 150°C durchgeführt werden; bevorzugt ist der Temperaturbreich von 30°C bis 100°C.

Die Chlorierungsreaktion kann so durchgeführt werden, daß man Allylisothiocyanat (II) mit dem gleichen bis zehnfachen seines Volumens mit dem Verdünnungsmittel mischt und in dem angegebenen Temperaturbereich mit Chlor umsetzt. Bevorzugt wird jedoch in er Weise verfahren, daß man in chlorgesättigtes Verdünnungsmittel unter stetigem weiteren Einleiten von überschüssigem Chlor (erkennbar an der grünen Farbe des Abgases), Allylsenföl (II) (gegebenenfalls in Mischung mit Verdünnungsmittel) in dem Maße zudosiert, daß zu jedem Zeitpunkt der Chlorierungsreaktion der Chlorüberschuß gewährt bleibt. Besonders bevorzugt ist es, diese geschilderte Art der Chlorierung bei der jeweiligen Siedetemperatur (unter Rückfluß) des Verdünnungsmittels, also beispielsweise im Falle von Chloroform bei ca. 60°C durchzuführen. Zur Erzielung einer möglichst vollständigen Umsetzung ist es außerdem zweckmäßig, nach beendeter Zudosierung (z.B. Zutropfen oder Zupumpen) des Allylisothiocyanats bei der gleichen Temperatur noch weitere 0,1 bis 2 Stunden nachzuchlorieren.

Das 2-Chlor-5-chlormethylthiazol (I) stellt bei den oben erwähnten, bevorzugten Reaktionsbedingungen das Hauptreaktionsprodukt dar. Außerdem ist als besonders vorteilhaft festzustellen, daß 2-Chlor-5-chlormethylthiazol (I) das nach erfolgter Umsetzung niedrigstsiedende Reaktionsprodukt darstellt und dadurch durch fraktionierende Destillation, z.B. an einer Kolonne, zweckmäßig im Vakuum (vorzugsweise Ölpumpe oder Wasserstrahlpumpe) leicht im Reinzustand erhalten werden kann. Zur Vermeidung unerwünschter thermischer Zersetzungsreaktionen ist es besonders vorteilhaft, die Abtrennung des 2-Chlor-5-chlormethylthiazols (I) von höhersiedenden Nebenreaktionsprodukten mittels eines zur Fraktionierung geeigneten Dünnschichtverdampfers vorzunehmen. Selbstverständlich lassen sich - insbesondere beim Arbeiten im Gramm-Maßstab - chromatographische Trennverfahren zur Reinisolierung von 2-Chlor-5-chlormethylthiazol (I) heranziehen.

Eine weitere Möglichkeit zur Reinisolierung von 2-Chlor-5-chlormethylthiazol (I) besteht darin, daß man zunächst auf das Rohgemisch nach beendeter Chlorierungsreaktion bei Raumtemperatur die halbe bis doppelte , vorzugsweise die gleiche Gewichtsmenge, bezogen auf eingesetztes Allylisothiocyanat, Ameisensäure bis zum Ende der Gasentwicklung einwirken läßt. Der Hauptanteil der höhersiedenden Nebenprodukte geht hierbei in schwerlösliche Feststoffe und/oder Öle über, die durch Filtration und/oder durch Dekantieren abgetrennt werden. Die überschüssige Ameisensäure wird vor der Destillation des 2-Chlor-5-chlormethylthiazols zweckmäßig durch Ausschütteln der organischen Phase mit Wasser oder durch Neutralisation mit der wäßrigen Lösung eines Alkali-oder Erdalkalihydroxids, vorzugsweise mit Natronlauge, entfernt.

2-Chlor-5-chlormethylthiazol (I) ist, wie in der veröffentlichten Europäischen Patentanmeldung Nr. 192 060 beschrieben, ein wichtiges Zwischenprodukt zur Herstellung von insektizid hochwirksamen Verbindungen, z.B. solchen mit den Formeln (III), (IV) und (V):

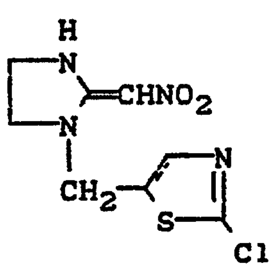

(III)

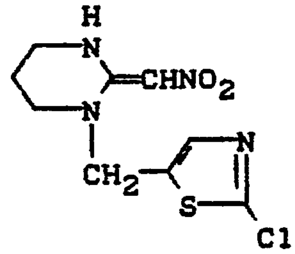

(IV)

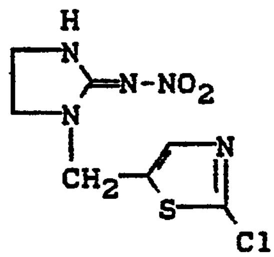

(V)

wobei zum Erhalt der o.g. Wirkstoffe (III), (IV) und (V) z.B. folgende Umsetzungen durchgeführt werden:

a)

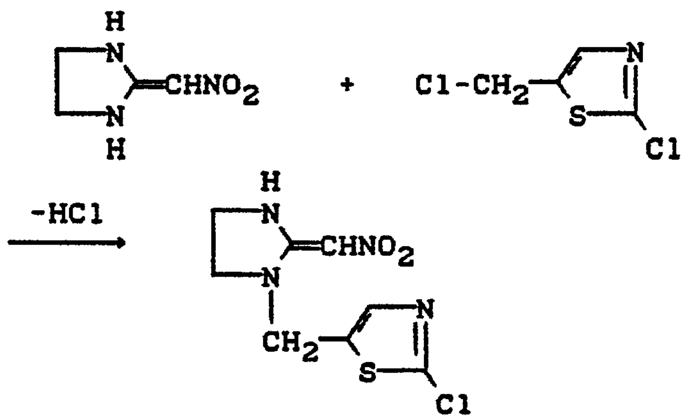


(III)

b)

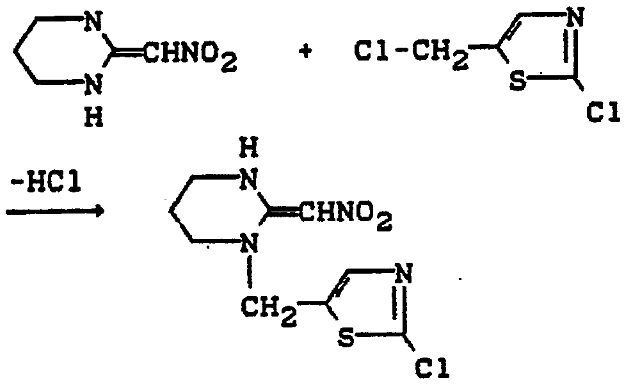


(IV)

c)

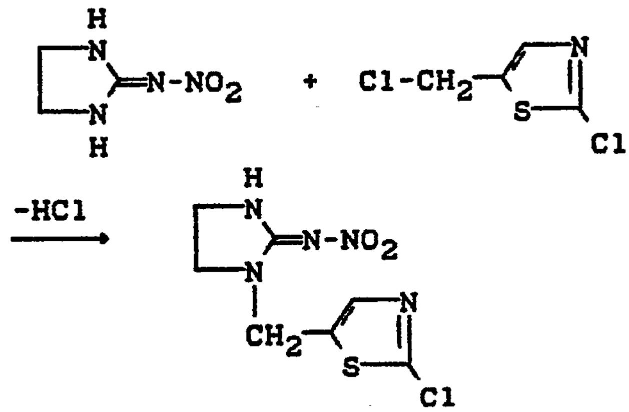


(V)

Beispiel 1

In 1,5 Liter unter Rückfluß (ca. 60°C) siedendes Chloroform wurden gleichzeitig ein stets überschüssiger Chlorstrom eingeleitet (erkennbar an der grünen Farbe des Abgases) sowie eine Mischung aus 500 g (4,54 Mol) 90 %igem Allylsenföl und 1 Liter Chloroform im Verlauf von etwa 5 Stunden zugepumpt. Chlorstrom und Allylsenföl-Zugabe wurden dabei so aufeinander abgestimmt, daß während der gesamten Reaktionsdauer im Abgas noch überschüssiges Chlor zu beobachten war. Nach beendetem Zupumpen des Allylsenföls wurde noch ca. 0,5 Stunden weiter Chlor eingeleitet. Die insgesamt durch die Reaktionsmischung geleitete Chlormenge betrug 3050 g. Nach Beendigung der Chlorzufuhr wurde in die weiterhin unter Rückfluß siedende Reaktionsmischung trockener Stickstoff zur Vertreibung von Chlor und Chlorwasserstoff geleitet, dann auf Raumtemperatur abgekühlt und anschließend im Rotationsverdampfer das Lösungsmittel im Wasserstrahlvakuum bis zu einer Badtemperatur von ca. 40°C entfernt. Man erhielt so 1222 g eines klaren, orangegelben Öls. Die gaschromatographische und massenspektroskopische Analyse ergab, daß eine Reaktionsgemisch vorlag, in welchem das niedrigstsiedende Reaktionsprodukt mit einem Prozentanteil von 41,1 % das Hauptprodukt darstellte. Es erwies sich als 2-Chlor-5-chlormethylthiazol, das in einer gaschromatographisch ermittelten Ausbeute von 65,8 % der Theorie entstanden war. Vorsichtige Destillation über eine einfache Destillationsbrücke an der Ölpumpe (zu Beginn und am Ende ca. 0,1 mbar, dazwischen Druckabfall bis ca. 1,5-2 mbar), bei einer Heizbadtemperatur von 100-140°C lieferte 805,5 g eines Destillats, bei dem nach der gaschromatographischen Analyse die niedrigstsiedende Verbindung mit einem Prozentanteil von 47,8 % das Hauptprodukt 2-Chlor-5-chlormethylthiazol (entsprechend 50,4 % der Theorie) darstellte. Fraktionierende Destillation an einer Kolonne von 150 cm Länge lieferte bei 50°C/1 mbar reines 2-Chlor-5-chlormethylthiazol.

$^1H$-NMR ($CDCl_3$; $\delta$ TMS = 0)

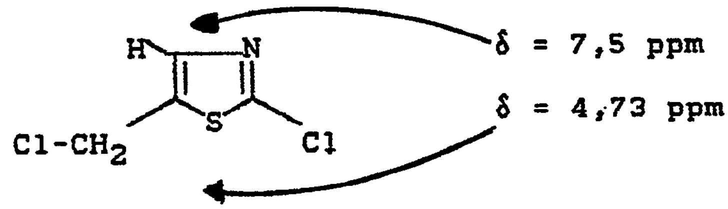


Die farblose reine Verbindung erstarrt bei Raumtemperatur zu langen Spießen.
Schmelzpunkt: 31°C.

Beispiel 2

Man arbeitete analog Beispiel 1 mit dem Unterschied, daß insgesamt 3940 g Chlorgas durch das Reaktionsgemisch geleitet wurden. Die gaschromatographische (GC)-Analyse des vom Lösungsmittel befreiten Rohproduktes (1250 g) ergab einen Prozentanteil von 41,3 % 2-Chlor-5-chlormethylthiazol, das entspricht einer GC-Ausbeute von 67,6 % der Theorie.

Beispiel 3

In 2,5 Liter unter Rückfluß siedendes Chloroform wurden gleichzeitig stets überschüssiges Chlor eingeleitet sowie eine Mischung aus 500 g (4,54 Mol) 90 %igem Allylsenföl und 0,5 Liter Chloroform im Zeitraum von 4,25 Stunden zugepumpt. Anschließend wurde noch ca. 0,5 Stunden weiter überschüssiges Chlor eingeleitet. Die insgesamt durch die Reaktionsmischung geleitete Chlormenge betrug 1990 g. Nach dem Austreiben von Chlor und Chlorwasserstoff durch Einleiten von trockenem Stickstoff wurden bei Raumtemperatur unter Rühren und Kühlen 500 ml reine Ameisensäure so zugetropft, daß sich die Temperatur der Reaktionsmischung zwischen 15 und 25°C hielt. Es wurde so lange in diesem Temperaturbereich weitergerührt, bis keine Gasentwicklung mehr zu beobachten war. Anschließend wurden unter Rühren und Kühlen bei etwa 20°C 500 ml Wasser zugetropft, von ausgefallenem Festprodukt abfiltriert bzw. von ausgeschiedenem, in der Chloroformphase unlöslichem Öl abdekantiert, die klare Chloroformphase abgetrennt und im Scheidetrichter fünfmal mit je 3 Liter Wasser bis zu einem pH-Wert von etwa 4 gewaschen. Anschließend wurde mit Natriumsulfat getrocknet und am Rotationsverdampfer das Chloroform abgezogen. Der Rückstand wurde im Wasserstrahlvakuum über eine einfache Destillationsbrücke grob fraktioniert. Bei einer Heizbadtemperatur von 120 bis 150°C und einem Druck zwischen 18 und 28 mbar wurden 331 g eines Destillats erhalten, das nach der gaschromatographischen Analyse zu 79,5 % (entsprechend 34,5 % der Theorie) aus 2-Chlor-5-chlormethylthiazol bestand. Feinfraktionierung an einer Kolonne lieferte reines 2-Chlor-5-chlormethylthiazol, identisch mit dem Produkt in Beispiel 1.

Beispiel 4

Man arbeitete zunächst analog Beispiel 1 mit dem Unterschied, daß einschließlich der Nachchlorierung innerhalb von 6,25 Stunden 3450 g Chlor durch das Reaktionsgemisch geleitet wurden. Anschließend wurde bei etwa 20°C mit 500 ml reiner Ameisensäure bis zum Ende der Gasentwicklung gerührt. Danach wurde unter Eiskühlung und unter gutem Rühren bei 0-10°C 22,5 %ige wäßrige Natronlauge bis zum pH = 7 zugetropft (Verbrauch etwa 2 550 ml), der ausgefallene schmierige Niederschlag abfiltriert, das Filtrat im Scheidetrichter in Chloroform- und Wasserphase getrennt, die Wasserphase noch zweimal mit Chloroform ausgeschüttelt und die vereinigten Chloroformphasen am Rotationsverdampfer eingeengt. Der Rückstand wurde im Wasserstrahlvakuum über eine einfache Destillationsbrücke grob fraktioniert. Bei einer Heizbadtemperatur von 90-120°C und einem Druck von etwa 20 mbar erhielt man einen Vorlauf von 92 g, der nach dem Gaschromatogramm (GC) 12,6 % (entsprechend einer Ausbeute von 1,5 % der Theorie) 2-Chlor-5-chlormethylthiazol enthielt. Der bei etwa 112°C/10 mbar destillierte Hauptlauf (220 g) war nach der gaschromatographischen Analyse 84,1 prozentiges 2-Chlor-5-chlormethylthiazol (entsprechend 24,2 % der Theorie). Feinfraktionierung an einer Kolonne lieferte reines 2-Chlor-5-chlormethylthiazol, identisch mit dem Produkt in Beispiel 1.

Beispiel 5

In 375 ml unter Rückfluß (ca. 40°C) siedendes Methylenchlorid wurden gleichzeitig ein stets überschüssiger Chlorstrom eingeleitet sowie eine Mischung aus 125 g (1,136 Mol) 90 %igem Allylsenföl und 250 ml Methylenchlorid im Verlauf von 1,25 Stunden zugepumpt. Nach beedetem Zupumpen des Allylsenföls wurde noch 0,5 Stunden weiter Chlor eingeleitet. Die insgesamt durch die Reaktionsmischung geleitete Chlormenge betrug 1020 g. Nach Beendigung der Chlorzufuhr wurde, wie in Beispiel 1 beschrieben, trockener Stickstoff durchgeleitet und anschließend das Lösungsmittel entfernt. Man erhielt 324 g eines klaren, gelben Öls, das nach der GC-Analyse zu 31,2 % aus 2-Chlor-5-chlormethylthiazol bestand. Daraus ergibt sich eine gaschromatographisch ermittelte Ausbeute von 53,0 % der Theorie.

Beispiel 6

In 3375 g (25 Mol) unter Rückfluß (ca. 69°C) siedendes Sulfurylchlorid wurden im Verlauf von etwa zwei Stunden 500 g (4,5 Mol) 90 %iges Allylisothiocyanat zugepumpt. Anschließend erhitzte man noch eine Stunde unter Rückfluß. Nach dem Abdestillieren des überschüssigen Sulfurylchlorids im Wasserstrahlvakuum am Rotationsver dampfer und weiterem Erwärmen bei 40°C bis 0,1 mbar (Ölpumpe) hinterblieben 1276,4 g eines gelben Öls, das nach der gaschromatographischen Analyse zu 33,6 % aus 2-Chlor-5-chlormethylthiazol bestand. Daraus ergibt sich eine gaschromatographisch ermittelte Ausbeute von 56,2 % der Theorie.

Beispiel 7

In 675 g (5 Mol) unter Rückfluß siedendes Sulfurylchlorid wurden im Verlauf von etwa 5,5 Stunden 100 g (0,9 Mol) 90 %iges Allylisothiocyanat zugepumpt. Anschließend erhitzte man noch eine Stunde unter Rückfluß. Das nach dem Abdestillieren des überschüssigen Sulfurylchlorids im Wasserstrahlvakuum am Rotationsverdampfer bis 40°C erhaltene Rohprodukt (262,6 g) wurde anschließend am Dünnschichtverdampfer bei 100-110°C/0,1 mbar destilliert. Das hellgelbe Destillat wog 189,4 g und enthielt nach der gaschromatograpischen Analyse 42,4 % 2-Chlor-5-chlormethylthiazol. Daraus ergibt sich eine gaschromatographisch ermittelte Ausbeute an destilliertem Produkt von 52,6 % der Theorie.

**Patentansprüche**

1. Verfahren zur Herstellung von 2-Chlor-5-chlormethylthiazol der Formel (I)

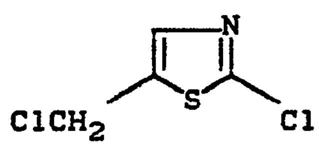

(I)

dadurch gekennzeichnet, daß man pro Mol Allylisothiocyanat der Formel (II)

$CH_2=CH-CH_2-NCS$ (II)

2 bis 20 Mol Chlor bei Temperaturen von 0°C bis 150°C gegebenenfalls in einem unter den Reaktionsbedingungen inerten Verdünnungsmittel einwirken läßt.

2. Verfahren nach Anspruch 1, dadurch gekennzeichnet, daß man mit elementaren Chlor oder mit einem elementares Chlor abspaltenden Chlorierungsmittel umsetzt.

3. Verfahren nach Anspruch 1 und 2, dadurch gekennzeichnet, daß man 5 bis 15 Mol Chlor bei 30 bis 100°C einwirken läßt.

4. Verfahren nach Anspruch 1 bis 3, dadurch gekennzeichnet, daß man als inertes Verdünnungsmittel Dichlormethan oder Trichlormethan verwendet.

5. Verfahren nach Anspruch 1 bis 4, dadurch gekennzeichnet, daß man in siedendes, stets überschüssiges Chlor enthaltendes Verdünnungsmittel Allylisothiocyanat zudosiert.

**Claims**

1. Process for the preparation of 2-chloro-5-chloro-methylthiazole of the formula (I)

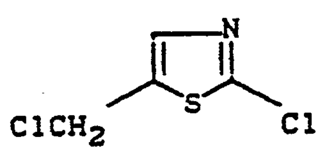

(I)

characterized in that 2 to 20 moles of chlorine are allowed to act on each mole of allyl isothiocyanate of the formula (II)

$CH_2=CH-CH_2-NCS$ (II)

at temperatures from 0°C to 150°C, if appropriate in a diluent which is inert under the reaction conditions.

2. Process according to Claim 1, characterized in that the reaction is carried out with elemental chlorine or with a chlorinating agent which dechlorinates to form elemental chlorine.

3. Process according to Claim 1 and 2, characterized in that 5 to 15 moles of chlorine are allowed to act at 30 to 100°C.

4. Process according to Claim 1 to 3, characterized in that the inert diluent used is dichloromethane or trichloromethane.

5. Process according to Claim 1 to 4, characterized in that allyl isothiocyanate is metered into a boiling diluent always containing chlorine in excess.

**Revendications**

1. Procédé de production de 2-chloro-5-chlorométhylthiazole de formule (I)

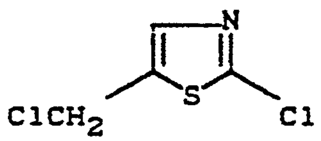

(I)

caractérisé en ce qu'on fait agir, par mole d'isothiocyanate d'allyle de formule (II)

$CH_2=CH-CH_2-NCS$ (II)

2 à 20 moles de chlore à des températures de 0 à 150°C, le cas échéant dans un diluant inerte dans les conditions réactionnelles.

2. Procédé suivant la revendication 1, caractérisé en ce qu'on conduit la réaction avec du chlore élémentaire ou avec un agent de chloration libérant du chlore élémentaire.

3. Procédé suivant les revendications 1 et 2, caractérisé en ce qu'on fait agir 5 à 15 moles de chlore à une température de 30 à 100°C.

4. Procédé suivant les revendications 1 à 3, caractérisé en ce qu'on utilise le dichlorométhane ou le trichlorométhane comme diluant inerte.

5. Procédé suivant les revendications 1 à 4, caractérisé en ce qu'on ajoute de l'isothiocyanate d'allyle au diluant en ébullition contenant du chlore toujours en excès.